# FASCICULE DE BREVET EUROPEEN

(11) **EP 1 450 474 B1**
(45) Date de publication et mention de la délivrance du brevet: **10.08.2011**
(21) Numéro de dépôt: 04290276.7
(22) Date de dépôt: 02.02.2004
(51) Int. Cl.: H02M 3/335

(54) **Convertisseur en transfert direct d'énergie**
Wandler und direkte Energieübertragung
Converter and direct energy transfer

(30) Priorité: 18.02.2003 FR 0301914
(43) Date de publication de la demande: 25.08.2004
(73) Titulaire: AEG Power Solutions B.V., 1161 AG Zwanenburg (NL)
(72) Inventeur: Diallo, Almadidi, 35235 Thorigne-Fouillard (FR); Puisieux, Philippe, 22300 Lannion (FR); Thereze, Jean-Marie, 22300 Lannion (FR)
(74) Mandataire: Thévenet, Jean-Bruno

(56) Documents cités:
- US-A- 5 430 640
- US-A- 6 061 255
- US-B1- 6 185 114

## Description

L'invention concerne un convertisseur en transfert direct d'énergie de type AC/DC ou DC/DC, comportant un redresseur synchrone autocommandé. Dans la suite du texte, l'expression « redresseur synchrone» désigne préférentiellement un redresseur synchrone autocommandé, c'est à dire un redresseur dans lequel les interrupteurs de redressement sont commandés à travers un ou plusieurs enroulements de transformateurs.

On connaît des systèmes de conversion en transfert direct d'énergie, comprenant une source de tension d'entrée et au moins un interrupteur primaire commandé, débitant dans un transformateur dont le secondaire est monté avec au moins deux interrupteurs de puissance constituant un redresseur synchrone autocommandé. Ledit redresseur synchrone est monté en cascade avec un filtre délivrant une tension continue contrôlée dans une application. Dans ce type de chaîne de conversion, le redresseur a pour but de
- délivrer à l'application, à travers le filtre, l'énergie transférée par le transformateur dans la période conductrice de l'interrupteur primaire, dite phase de transfert direct, et
- bloquer le transfert dans la période non-conductrice dudit interrupteur primaire, l'application étant alimentée par la bobine du filtre, à travers un interrupteur de roue libre tel qu'un transistor MOSFET du redresseur, durant cette période non-conductrice de l'interrupteur principal primaire, dite phase de roue libre.

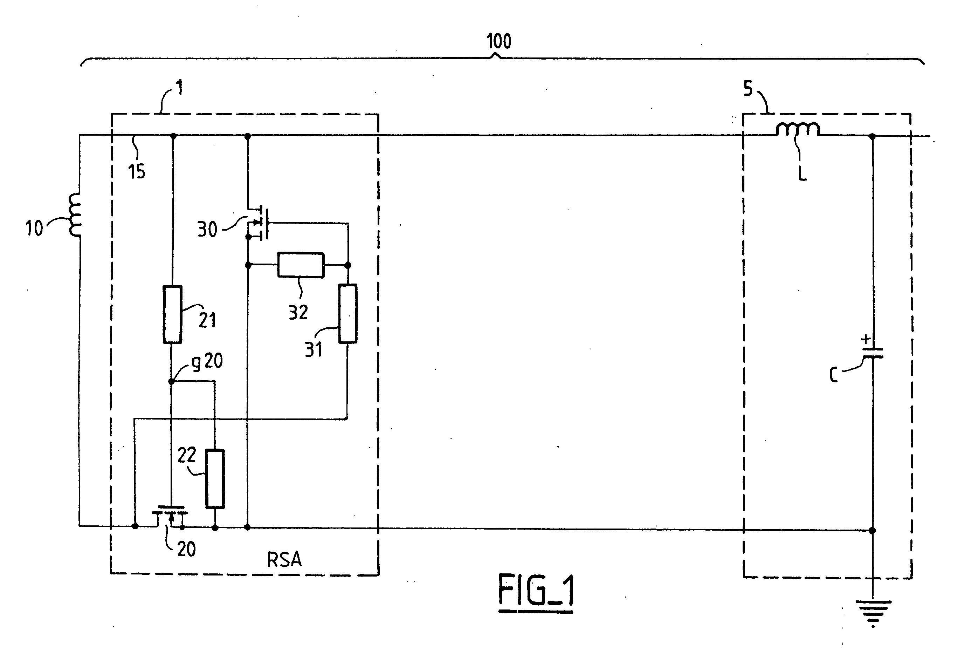

La partie secondaire 100 d'un tel convertisseur, du type à transfert direct d'énergie asymétrique, est représentée en figure 1. Le redressement est dit à écrêtage actif (« active clamp » en anglais).

La partie 100 comporte :
- un enroulement secondaire 10 de transformateur de puissance,
- un redresseur synchrone 1 de type à transfert direct,
- un filtre de sortie LC 5.

Le redresseur synchrone 1 comporte deux transistors de puissance secondaires 20 et 30, et leurs éléments de commande respectifs 21-22 et 31-32, agencés de manière à assurer le transfert direct d'énergie par le transistor 20 et la phase de roue libre réalisée par le transistor 30 avec le minimum de pertes. Les éléments 21 et 31 sont par exemple une diode en parallèle avec une capacité et les éléments 22 et 32 sont par exemple des diodes Zener.

Cette solution n'est possible que par une optimisation de la tension de commande des grilles. Le câblage des éléments de commande en auto-commandé donne des tensions sur les grilles des transistors de puissance dont les amplitudes dépendent directement de la tension d'entrée aux bornes de l'enroulement primaire non représenté et donc de la tension de sortie aux bornes de l'enroulement secondaire 10. Dans les systèmes à transfert direct d'énergie asymétriques de type à écrêtage actif, les amplitudes des tensions de grille varient en sens inverse. Par exemple, la tension disponible sur la grille du transistor 20, dans la phase directe, est maximale pour une tension d'entrée maximale. Inversement, lorsque la tension d'entrée diminue la tension disponible sur la grille du transistor 30 augmente pour atteindre une valeur maximale qui peut être inadaptée.

Il en résulte que cette solution pose un certain nombre de difficultés. Il se peut en effet que la tension maximale de grille de l'un des deux transistors ne soit pas suffisante pour réaliser la commande. C'est le cas notamment lorsque la tension aux bornes de l'enroulement secondaire 10 est faible, de l'ordre de 2V. Une tension faible aux bornes de l'enroulement secondaire peut notamment se produire dans le cas d'une tension d'entrée variable aux bornes de l'enroulement primaire, par exemple entre 36V et 72V pour une tension moyenne de 48V ou entre 18V et 36V pour une tension moyenne de 24V et davantage encore pour de plus larges plages de tension d'entrée telles que 18V - 72V. La tension aux bornes de l'enroulement secondaire peut alors être juste suffisante pour commander le transistor 20 dans la phase directe mais insuffisante pour commander le transistor 30 dans la phase de roue libre.

A l'inverse, pour des tensions de sorties fortes, supérieures à 10V, il n'est pas aisé de trouver un bon compromis entre une tension de grille suffisante sur le transistor direct, dans toute la plage de tension d'entrée, et une tension de grille maximale mais inférieure à la valeur maximale admissible pour la grille du transistor de roue libre.

On connaît aussi avec le brevet US5430640 un convertisseur d'énergie dont le commutateur de roue libre est alimenté depuis une source de tension auxiliaire. Toutefois, cette architecture ne permet pas d'anticiper le changement de phase dans le signal de commande et donc ne minimise pas les pertes au maximum. Notamment, dans certains cas d'utilisation, il faut avoir recours à un circuit d'écrêtage pour limiter les pics de tension sur les transistors de redressement synchrone pour éviter l'avalanche de ces derniers.

La présente invention vise à réaliser un convertisseur en transfert direct d'énergie en redressement synchrone auto-commandé, notamment du type à écrêtage actif, permettant de supprimer aussi bien les pertes de conduction, dues à l'inadéquation des tensions de commande lorsque la tension de sortie est faible que les pertes de commutation générées quand la tension de sortie est élevée, en garantissant une tension de commande suffisante dans la phase de roue libre et dans la phase directe.

La présente invention propose à cet effet un convertisseur en transfert direct d'énergie selon la revendication 1.

Grâce à l'invention, la commande du deuxième interrupteur pour passer en phase de roue libre est indépendante de la tension aux bornes de l'enroulement secondaire durant la phase de roue libre. Ainsi, une variation de la tension d'entrée qui entraînerait, dans la phase de roue libre, une baisse de la tension aux bornes de l'enroulement secondaire n'a aucune influence sur la commande de l'interrupteur secondaire de roue libre.

De manière avantageuse, la source de tension auxiliaire sera réalisée par un circuit aussi simple que possible, mais à condition qu'il puisse fournir une tension convenable dans tous les cas, selon le contexte d'utilisation du convertisseur.

Ainsi, pour des raisons de compacité et de coût, il sera généralement préférable que la source de tension auxiliaire prélève son énergie du convertisseur lui-même. Celle-ci pourrait être prélevée en sortie du convertisseur, c'est-à-dire en aval du filtre de sortie.

Néanmoins une solution particulièrement intéressante dans le cas où la tension de sortie du convertisseur serait susceptible de devenir trop basse consiste à ce que la source de tension auxiliaire puisse fournir une tension sensiblement égale à la tension de l'enroulement secondaire pendant la phase directe. Ainsi, la source de tension auxiliaire est un circuit conçu pour emmagasiner de l'énergie fournie par l'enroulement secondaire, sous une tension sensiblement égale à la tension de crête disponible à ses bornes. Cette disposition permet de produire une tension supérieure à celle que l'on obtient en sortie du convertisseur car cette dernière n'est que la tension moyenne de la tension présente aux bornes de l'enroulement secondaire.

Ainsi, la tension de l'enroulement secondaire est choisie pour optimiser la commande du premier interrupteur transistor de transfert direct d'énergie. La tension de commande du deuxième interrupteur permettant l'entrée en phase de roue libre est directement liée à la tension de commande permettant l'entrée en phase de transfert direct d'énergie. Le convertisseur selon l'invention permet donc d'optimiser la commande du premier interrupteur sans se soucier de la commande du deuxième interrupteur.

Selon une deuxième variante, ledit convertisseur comportant des seconds moyens auto-commandés pour appliquer une tension de commande qui permet de rendre passant ledit premier interrupteur en phase directe, cette tension de commande est fournie par ladite source de tension auxiliaire par l'intermédiaire d'un second circuit de transfert de charge.

Ainsi, cette deuxième variante permet de commander les deux interrupteurs du redresseur par une tension de commande choisie, par exemple à partir d'une tension sensiblement égale à la tension de l'enroulement secondaire pendant la phase directe.

Selon un mode de réalisation particulier, ladite source de tension auxiliaire comporte :
- une capacité,
- un élément redresseur relié par sa première extrémité en série avec ladite capacité et par sa deuxième extrémité à une extrémité dudit enroulement secondaire, ledit élément redresseur réalisant une tension auxiliaire sensiblement égale à la tension de l'enroulement secondaire pendant la phase directe.

Avantageusement, une diode est montée en anti-parallèle entre la base et l'émetteur dudit transistor bipolaire de charge.

De manière particulièrement avantageuse, le convertisseur comporte des moyens pour anticiper le blocage dudit deuxième interrupteur avant la mise en conduction dudit premier interrupteur dans ladite phase directe.

Selon un mode de réalisation particulier lesdits moyens pour anticiper le blocage dudit deuxième interrupteur avant la mise en conduction dudit premier interrupteur dans ladite phase directe comportent :
- un réseau dérivateur comprenant un condensateur relié en série à une résistance, ladite résistance étant reliée à une extrémité dudit enroulement secondaire, ledit réseau détectant la variation de la tension induite par ledit enroulement primaire aux bornes dudit enroulement secondaire,
- un transistor bipolaire, dit transistor d'anticipation, la base dudit transistor bipolaire étant commandée via ledit réseau dérivateur, l'émetteur dudit transistor bipolaire étant relié au drain dudit premier interrupteur et le collecteur dudit transistor bipolaire étant relié à la grille dudit deuxième interrupteur.

Avantageusement, selon ce dernier mode de réalisation particulier, une diode est montée en anti-parallèle entre la base et l'émetteur dudit transistor bipolaire d'anticipation.

Avantageusement, ledit convertisseur comporte des moyens pour retarder la mise en conduction dudit premier interrupteur tant que ledit deuxième interrupteur est commandé.

De manière avantageuse, lesdits moyens pour retarder la mise en conduction dudit premier interrupteur tant que ledit deuxième interrupteur est commandé comportent :
- un transistor MOSFET, dit transistor de retardement,
- une résistance ayant une première borne reliée à la grille dudit transistor de roue libre et une deuxième borne reliée à la grille dudit transistor de retardement,
- une capacité ayant une première borne reliée à la grille dudit transistor de retardement et une deuxième borne reliée à la source dudit transistor direct.

De manière avantageuse, ledit étage primaire et ledit étage secondaire sont isolés galvaniquement.

Ainsi, le circuit conserve une isolation galvanique très souvent indispensable dans ce type de convertisseur pour des raisons de sécurité.

D'autres caractéristiques et avantages de la présente invention apparaîtront dans la description suivante de modes de réalisation de l'invention, donnés à titre illustratif et nullement limitatif.

Dans les figures suivantes :
- La figure 1 représente schématiquement la partie secondaire d'un convertisseur en transfert direct d'énergie selon l'art antérieur,
- La figure 2 représente schématiquement la partie secondaire d'un convertisseur en transfert direct d'énergie selon un premier mode de réalisation de l'invention,
- La figure 3 représente les diagrammes de tension en fonction du temps relativement à un convertisseur ayant une partie secondaire telle que représentée en figure 2.
- La figure 4 représente schématiquement la partie secondaire d'un convertisseur en transfert direct d'énergie selon un deuxième mode de réalisation de l'invention,
- La figure 5 représente schématiquement la partie secondaire d'un convertisseur en transfert direct d'énergie selon un troisième mode de réalisation de l'invention.

Dans toutes les figures, les éléments communs portent les mêmes numéros de référence.

La figure 1 a déjà été décrite en relation avec l'état de la technique.

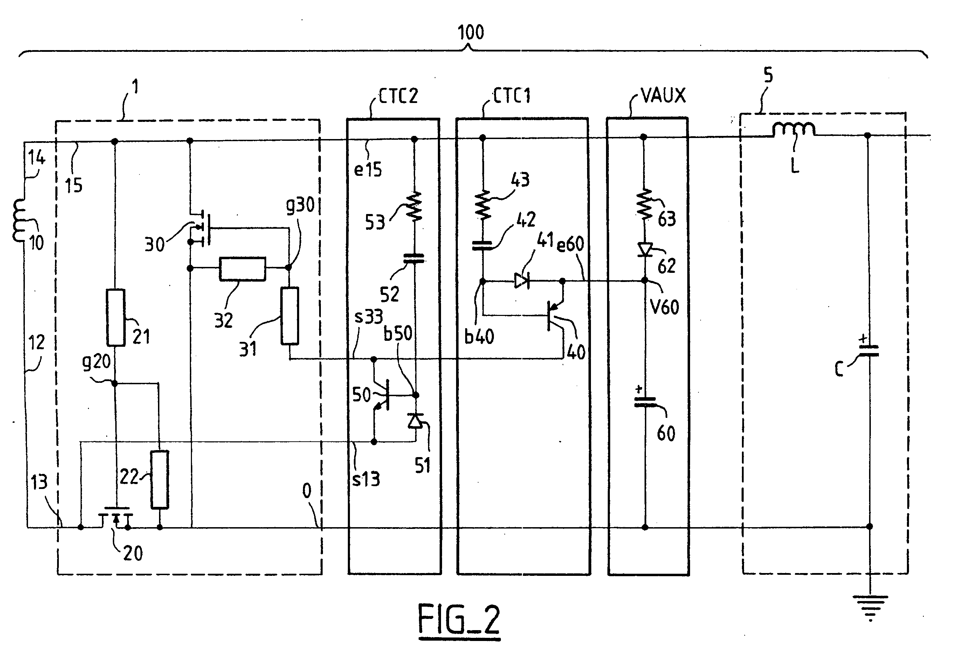

La figure 2 représente la partie secondaire 100 d'un convertisseur 100 en transfert direct d'énergie selon un premier mode de réalisation de l'invention. Notons que la partie primaire du convertisseur, non représenté ici, est réalisée de façon connue grâce à un enroulement primaire de transformateur de puissance câblé à une source de tension d'entrée, au moyen d'éléments de commutation montés préférentiellement en transfert direct d'énergie avec écrêtage actif.

La partie 100 comporte :
- un enroulement secondaire 10 de transformateur de puissance,
- un redresseur synchrone 1 de type à transfert direct d'énergie,
- un filtre de sortie LC 5,
- des moyens CTC1, dit premier circuit de transfert de charge,
- des moyens CTC2, dit deuxième circuit de transfert de charge, une source de tension auxiliaire VAUX.

Le redresseur synchrone 1 comporte deux interrupteurs secondaires qui sont deux transistors MOSFET de puissance 20 (MOSFET direct) et 30 (MOSFET de roue libre) assurant respectivement les phases directe et de roue libre du redresseur 1.

La grille du transistor de puissance 20 assurant le transfert direct d'énergie est commandée par l'enroulement de puissance secondaire 10 via les impédances 21 et 22, l'impédance 21 étant reliée entre la grille du transistor 20 et l'extrémité 15 de l'enroulement 10 et l'impédance 22 étant reliée à la grille et à la source du transistor 20.

Les moyens CTC1 et CTC2 sont des moyens de contrôle du transistor MOSFET de roue libre 30 et seront décrits plus précisément par la suite.

L'enroulement secondaire de puissance 10 comporte :
- une première extrémité 14 reliée à l'entrée commune 15 du redresseur 1 et du filtre de sortie 5 et à l'entrée e15 des circuits de transfert de charge CTC1 et CTC2,
- une seconde extrémité 12 reliée en série à l'entrée 13 du redresseur 1.

Les entrées 15 et 13 dudit redresseur 1 sont reliées respectivement aux transistor MOSFET 30 et 20 assurant les phases de roue libre et de transfert direct d'énergie.

Les tensions de l'étage secondaire sont référencées par rapport aux sources des interrupteurs de puissance 20 et 30 reliées au point 0 du redresseur 1. Le point 0 fait donc office de masse de la partie secondaire 100.

La source de tension auxiliaire VAUX, disponible au point V60, comporte en série une résistance 63, une diode 62 et une capacité de charge 60, cet ensemble en série étant relié entre l'extrémité 14 de l'enroulement 10 et la masse du point 0.

Les moyens CTC1 comportent un transistor 40, dit transistor de charge, de type transistor bipolaire pnp de signal ; notons que le transistor 40 peut être un transistor MOS de signal canal P, mais seule la réalisation des moyens CTC1 avec un transistor bipolaire type pnp sera décrite.

Le collecteur du transistor bipolaire pnp de signal 40 est relié à la grille g30 du transistor de roue libre 30 via une impédance 31 optionnelle, l'impédance 31 étant assimilée à un court-circuit dans la suite du document.

La base du transistor bipolaire 40 est commandée via un réseau dérivateur RC constitué d'un condensateur 42 en série avec une résistance 43 reliée à l'extrémité commune e15 des moyens CTC1 et CTC2, l'extrémité e15 étant elle-même reliée à l'extrémité 15 de l'enroulement secondaire 10 réalisant une commande du transistor 40, synchronisée par le signal de tension au point e15, noté V(e15), permettant d'assurer le transfert de charge de la source VAUX, disponible en v60 aux bornes de l'élément 60, vers la grille de l'interrupteur de puissance 30 durant la phase de roue libre pendant laquelle le signal V(e15) est quasi-nul. Cette charge est unidirectionnelle et reste mémorisée sur la grille dans la phase de roue libre. Les moyens CTC1 sont donc des moyens pour rendre passant ledit interrupteur de roue libre 30 indépendamment de la tension aux bornes de l'enroulement secondaire 10 pendant la phase de roue libre.

L'émetteur du transistor bipolaire 40 est relié à la source de tension continue VAUX obtenue ici par le redressement de la tension disponible sur l'extrémité 15 de l'enroulement secondaire 10 grâce à l'élément redresseur 62 et la résistance optionnelle 63. En choisissant la tension VAUX comme décrite, on obtient une tension de grille du transistor MOSFET 30 de roue libre variant dans le même sens et équivalente à celle disponible sur la grille du transistor MOSFET direct 20, et par conséquent, proportionnelle à la tension d'entrée. La tension VAUX peut néanmoins être choisie fixe par rapport à la tension de l'enroulement secondaire 10 pendant la phase directe et donc par rapport à la tension d'entrée ou variant avec la tension d'entrée dans des proportions acceptables pour la grille commandée.

Dans la phase directe, la tension V(e15) devient positive. La diode 41 montée en anti-parallèle sur la base du transistor 40, entre les points b40 et v60, autorise un courant inverse dans le réseau dérivateur RC 42-43 afin de permettre la synchronisation et le transfert de charge de la source VAUX vers la grille du transistor de puissance 30 dans la phase de roue libre.

Le circuit CTC2 comporte un transistor bipolaire de signal 50, dit transistor d'anticipation, de type npn, permettant d'anticiper le blocage du transistor de roue libre 30 dès le changement de pente de la tension aux bornes de l'enroulement secondaire 10. En effet, le changement de pente est détecté par le réseau dérivateur RC constitué du condensateur 52 et de la résistance 53, ces deux éléments étant montés en série puis câblés entre la base b50 du transistor 50 et l'entrée e15, l'entrée e15 étant elle-même reliée à l'extrémité 15 de l'enroulement secondaire 10.

L'émetteur du transistor d'anticipation 50 est reliée au drain du transistor de puissance 20 assurant le transfert direct d'énergie dans la phase directe, le drain du transistor de puissance 20 étant lui-même relié à l'entrée 13 du redresseur 1, l'entrée 13 étant elle-même câblée sur la sortie 12 de l'enroulement secondaire 10. Le collecteur du transistor d'anticipation 50 est relié à la grille g30 du transistor de roue libre 30 à travers une impédance 31 assimilable à un court-circuit et le collecteur est aussi raccordé à celui du transistor 40 des moyens CTC1 formant la jonction s33.

Dans la phase de roue libre, la tension aux bornes de l'enroulement secondaire 10 s'inverse, la tension V(e15) devient quasi-nulle, la diode 51 monté en anti-parallèle sur la jonction base-émetteur du transistor d'anticipation 50, entre les points13 et b50, autorise un courant inverse dans le réseau dérivateur RC 52-53 afin de permettre la synchronisation et l'anticipation d'élimination de la charge de la grille du transistor de puissance 30 juste avant la phase active de transfert direct d'énergie. Les moyens CTC2 sont donc des moyens pour anticiper le blocage de l'interrupteur 30 de roue libre avant la mise en conduction de l'interrupteur 20 de transfert d'énergie direct dans la phase directe.

Dans cette première réalisation, la grille du transistor de puissance 20 assurant le transfert direct d'énergie est commandée par l'enroulement de puissance secondaire 10 et comporte des moyens connus 21-22 de commande et protection en redressement synchrone auto-commandé.

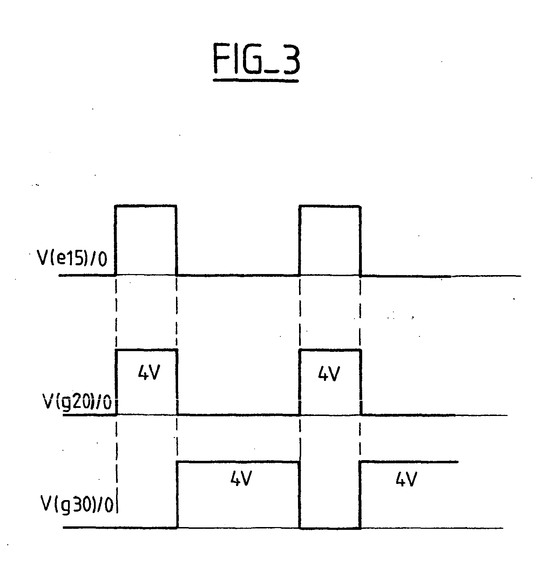

La figure 3 représente les diagrammes V(e15), V(g20) et V(g30) de tension en fonction du temps relativement à un convertisseur ayant une partie secondaire telle que représentée en figure 2.

V(e15) est le signal de tension aux points 15 du redresseur 1 confondu avec le point e15 d'extrémité commune des moyens CTC1 et CTC2 en fonction du temps et référencé au potentiel des sources communes des transistors de puissance 20 et 30, reliées à la masse 0 du secondaire.

V(g20) et V(g30) sont les tensions de grille des transistors 20 et 30 référencées aux sources communes 0 ou masse du secondaire.

Dans la phase de pente positive de la tension d'enroulement de puissance secondaire 10, précédant le palier positif du signal V(e15), le réseau dérivateur RC 52-53 génère un courant direct dans la base du transistor de signal 50 qui se met à conduire pour annuler la charge de grille du transistor MOSFET de puissance 30. Le canal du transistor MOSFET 30 se trouve bloqué et le courant de roue-libre passe alors dans la diode parasite du transistor MOSFET 30 ou dans une diode externe montée en parallèle. La tension d'enroulement continue de croître et génère une charge positive sur la grille du transistor MOSFET direct 20 et la résistance du canal du transistor MOSFET 20 devient quasi-nulle dans le palier positif du signal V(e15). Le signal V(e15) est alors égal à la tension aux bornes de l'enroulement de puissance secondaire 10.

Dans le palier positif du signal V(e15), le transistor 40 de charge est bloqué. La diode 41 permet de générer un courant dit négatif dans le réseau dérivateur RC 42-43 afin de permettre la synchronisation du transfert de charge de l'alimentation auxiliaire VAUX disponible en v60 vers la grille du transistor MOSFET 30 dans la phase suivante dite de roue-libre ; ce courant dans la diode 41 et le courant disponible dans la diode d'alimentation 62 chargent le condensateur 60 d'alimentation auxiliaire VAUX à une tension voisine de la tension d'enroulement disponible, en g20, sur la grille du transistor MOSFET direct 20.

La phase directe s'achève par un changement de pente initié par le circuit de contrôle primaire. La pente négative qui en résulte, aux bornes de l'enroulement 10 et en V(e15), entraîne à travers la diode 51 un courant inverse dans le réseau dérivateur RC 52-53 compensant le courant positif induit dans la phase de pente positive précédente. Le transistor d'anticipation 50 est bloqué, autorisant de ce fait le transfert de charge de VAUX vers la grille du transistor de puissance 30 à travers le transistor de charge 40 dès que la tension disponible en V(e15) autorise une polarisation directe de sa base, cela grâce au réseau dérivateur RC 42-43 traversé par le courant de base du transistor 40, en opposition du courant dans la diode 41 lors de la phase précédente. La charge de grille disponible en g30 est ainsi quasi égale à celle qui était disponible en g20 ; elle sera maintenue durant tout le palier où le signal V(e15) est quasi-nul, c'est à dire la phase de roue-libre.

La phase de roue libre se termine par un changement de pente sur la tension aux bornes de l'enroulement de puissance 10 initié par le circuit de contrôle primaire. La pente positive qui en résulte anticipe le blocage du transistor de puissance 30 en générant dans la base du transistor d'anticipation 50 un courant direct dans le réseau RC 52-53, comme décrit en début de cycle.

Ainsi, les deux transistors MOSFET 20 et 30 sont commandés par une tension de grille quasi identique et variant dans le même sens que la tension d'entrée dont l'image dans l'étage secondaire est le signal V(e15) disponible sur l'enroulement 10 pendant la phase directe.

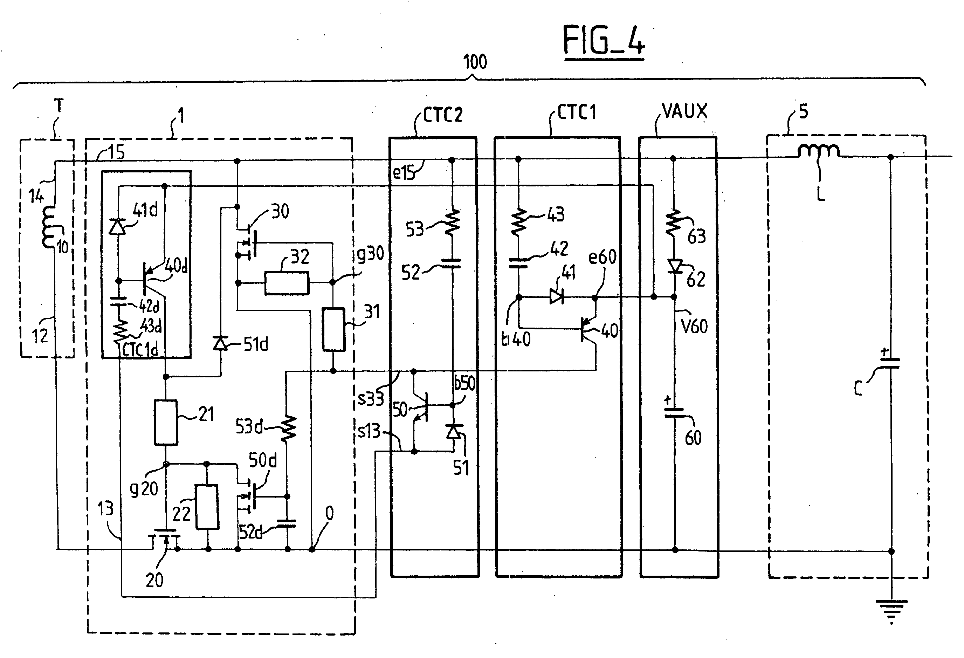

La figure 4 représente schématiquement la partie secondaire 100 d'un convertisseur en transfert direct d'énergie selon un deuxième mode de réalisation de l'invention.

Cette partie secondaire 100 est identique à celle représentée en figure 2 à la différence qu'elle comporte deux autres circuits de transfert de charge CTC1d et CTC2d afin de réaliser la commande de la grille du transistor direct 20 qui dépende uniquement de la source de tension VAUX.

Le circuit CTC1d est réalisé de manière identique au circuit CTC1 et comporte:
- un transistor bipolaire 40d,
- une résistance 42d,
- une capacité 42d,
- une diode 41d.

Le circuit CTC2d comporte :
- un transistor MOSFET 50d, dit transistor de retardement,
- une capacité 52d,
- une résistance 53d,
- une diode 51d.

Le circuit CTC1d fonctionne de manière identique au circuit CTC1 et assure la charge de la grille du transistor direct 20.

La résistance 53d a une première borne reliée à la grille du transistor de roue libre 30 via la résistance 31 et une deuxième borne reliée à la grille du transistor de retardement 50d.

La capacité 52d a une première borne reliée à la grille du transistor de retardement 50d et une deuxième borne reliée à la source du transistor direct 20.

Concernant le circuit CTC2d, la diode 51d assure la décharge de la grille du transistor direct 20. Le transistor MOSFET 50d, la capacité 52d associés à la résistance 53d retardent la montée de la charge de la grille du transistor 20 tant qu'une charge est présente sur la grille du transistor 30.

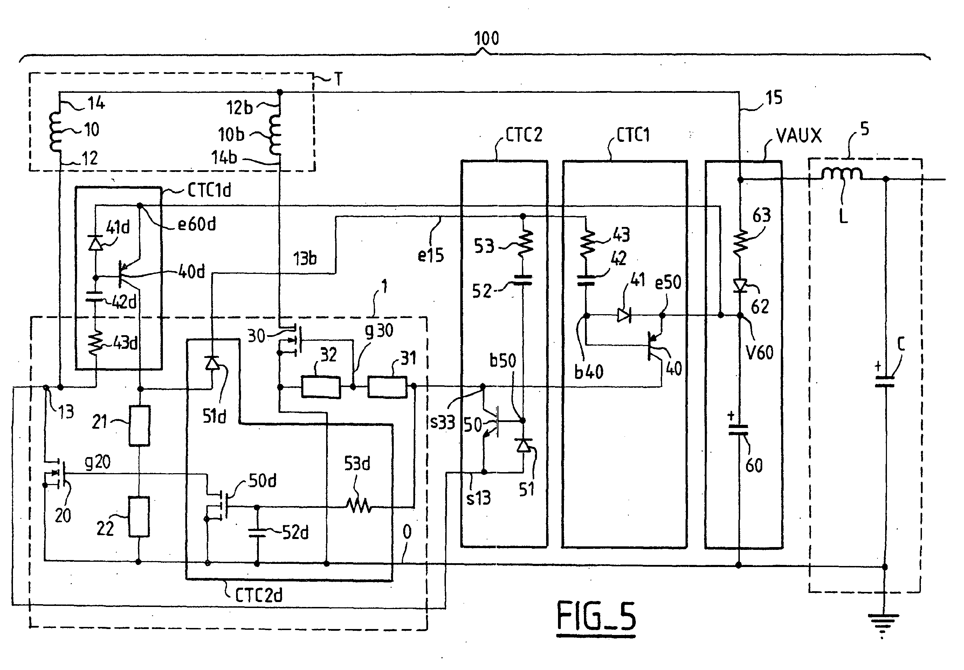

La figure 5 représente schématiquement la partie secondaire 100 d'un convertisseur en transfert direct d'énergie selon un troisième mode de réalisation de l'invention.

La partie secondaire 100 comporte ici deux enroulements 10 et 10b de puissance câblés en opposition de phase sur les transistors de redressement 20 et 30 et réalise ainsi un redressement synchrone asymétrique dit « double alternance » avec écrêtage actif. Les circuits CTC1, CTC2, CTC1d et CTC2d sont réalisés de manière identique aux circuits représentés en figure 4 mais les signaux de synchronisation de ces circuits changent car le signal disponible au point 15, point commun des deux enroulements 10 et 10b, est quasi continu.

Ainsi, le signal de synchronisation des circuits CTC1 et CTC2 est pris au point 13b reliant le drain du transistor 30 à l'extrémité 14b de l'enroulement 10b.

De même, l'élément d'aide au blocage 51 d du transistor 20 est raccordé au point 13b.

Comme pour les figures 2 et 4, les circuits de transfert de charge CTC1, CTC2 et CTC1d sont synchronisés par les signaux variables d'enroulement du transformateur. Les circuits CTC1 et CTC1d assurent une commande de la grille de chacun des transistors 20 et 30 proportionnelle à la tension d'entrée. Notons à nouveau que cette tension de commande peut également être indépendante de la tension d'entrée si on prend une tension VAUX indépendante.

Bien entendu, l'invention n'est pas limitée aux modes de réalisation qui viennent d'être décrits.

Notamment, les transistors dits de charge et d'anticipation ont été décrits comme des transistors bipolaires mais il peut également s'agir de transistor MOSFET de signal.

## Revendications

1. Convertisseur en transfert direct d'énergie comportant :
- un étage primaire comportant au moins un enroulement primaire d'un transformateur et au moins un interrupteur commandé qui présente des phases de fonctionnement conductrices et non-conductrices,
- un étage secondaire comportant au moins un enroulement secondaire (10) dudit transformateur et un redresseur synchrone (1) comportant :
o au moins un premier transistor MOSFET (20), dit interrupteur direct, auto-commandé et passant pendant les phases conductrices dudit interrupteur commandé de l'étage primaire, dites phases de transfert direct de l'énergie,
o au moins un deuxième transistor MOSFET (30), dit interrupteur de roue libre, auto-commandé et passant pendant les phases non-conductrices dudit interrupteur commandé de l'étage primaire, dites phases de roue libre,
- un filtre de sortie (5),
ledit convertisseur comportant des premiers moyens auto-commandés, déclenchés en fonction de la tension aux bornes dudit au moins un enroutement secondaire (10) et appliquant audit deuxième transistor MOSFET (30) une tension de commande correspondante apte à rendre passant ledit deuxième interrupteur (30),
lesdits premiers moyens auto-commandés comportent un premier circuit de transfert de charge (CTC1) commandé directement par ladite tension aux bornes dudit enroulement secondaire (10) pour appliquer audit deuxième interrupteur (30) une tension de commande sensiblement constante et fournie par une source de tension auxiliaire (VAUX), **caractérisé en ce que** ledit au moins un enroulement secondaire est connecté en parallèle au redresseur synchrone (1), et ledit premier circuit de transfert de charge (CTC1) comporte :
- un réseau dérivateur comprenant un condensateur (42) relié en série avec une résistance (43), ladite résistance étant reliée à une extrémité (14) dudit au moins un enroulement secondaire (10), ledit réseau détectant la variation de la tension induite par ledit au moins un enroulement primaire aux bornes dudit au moins un enroulement secondaire (10),
- un transistor bipolaire (40), dit transistor de charge, la base dudit transistor bipolaire étant commandée via ledit réseau, l'émetteur dudit transistor bipolaire étant relié à ladite source de tension auxiliaire et le collecteur dudit transistor bipolaire étant relié à la grille dudit deuxième transistor MOSFET (30).

2. Convertisseur selon la revendication 1 **caractérisé en ce que** ladite source de tension auxiliaire (VAUX) fournit une tension (V60) sensiblement égale à la tension dudit enroulement secondaire (10) au moins un pendant la phase directe.

3. Convertisseur selon la revendication 2 **caractérisé en ce que** ladite source de tension auxiliaire (VAUX) comporte :
- une capacité (60),
- un élément redresseur (62) relié par sa première extrémité en série avec ladite capacité (60) et par sa deuxième extrémité à une extrémité (15) dudit au moins un enroulement secondaire (10), ledit élément redresseur réalisant une tension auxiliaire sensiblement égale à la tension dudit au moins un enroulement secondaire (10) pendant la phase directe.

4. Convertisseur selon l'une des revendications 1 à 3 **caractérisé en ce que** la tension de commande dudit premier transistor MOSFET (20) est obtenue à partir de la tension aux bornes dudit au moins un enroulement secondaire (10).

5. Convertisseur selon l'une des revendications 1 à 3 **caractérisé en ce que** ledit convertisseur comportant des seconds moyens auto-commandés pour appliquer une tension de commande qui permet de rendre passant ledit premier transistor MOSFET (20) en phase directe, cette tension de commande est fournie par ladite source de tension auxiliaire (VAUX) par l'intermédiaire d'un second circuit de transfert de charge (CTC1d).

6. Convertisseur selon la revendication 5 **caractérisé en ce qu'**une diode (41) est montée en anti-parallèle entre la base et l'émetteur dudit transistor bipolaire de charge (40).

7. Convertisseur selon l'une des revendications 1 à 6 **caractérisé en ce qu'**il comporte des moyens (CTC2) pour anticiper le blocage dudit deuxième transistor MOSFET (30) avant la mise en conduction dudit premier transistor MOSFET (20) dans ladite phase directe.

8. Convertisseur selon la revendication 7 **caractérisé en ce que** lesdits lesdits moyens (CTC2) pour anticiper le blocage dudit deuxième transistor MOSFET (30) avant la mise en conduction dudit premier transistor MOSFET (20) dans ladite phase directe comporte :
- un réseau dérivateur comprenant un condensateur (52) relié en série avec une résistance (53), ladite résistance étant reliée à une extrémité (14) dudit au moins un enroulement secondaire (10), ledit réseau détectant la variation de la tension induite par ledit enroulement primaire aux bornes dudit enroulement secondaire (10),
- un transistor bipolaire (50), dit transistor d'anticipation, la base dudit transistor bipolaire étant commandée via ledit réseau dérivateur, l'émetteur dudit transistor bipolaire étant relié au drain dudit premier interrupteur et le collecteur dudit transistor bipolaire étant relié à la grille dudit deuxième interrupteur.

9. Convertisseur selon la revendication 8 **caractérisé en ce qu'**une diode (51) est montée en anti-parallèle entre la base et l'émetteur dudit transistor bipolaire d'anticipation.

10. Convertisseur selon la revendications 4 **caractérisé en ce que** ledit second circuit de transfert de charge (CTC1d) comporte :
- un réseau dérivateur comprenant un condensateur (42d) relié en série à une résistance (43d), ladite résistance étant reliée à une extrémité dudit au moins un enroulement secondaire (10), ledit réseau détectant la variation de la tension induite par ledit au moins un enroulement primaire aux bornes dudit au moins un enroulement secondaire (10),
- un transistor bipolaire (40d), dit transistor de charge, la base dudit transistor bipolaire étant commandée via ledit réseau, l'émetteur dudit transistor bipolaire étant relié à ladite source de tension auxiliaire (VAUX) et le collecteur dudit transistor bipolaire étant relié à la grille dudit premier transistor MOSFET (20)

11. Convertisseur selon l'une des revendications 1 à 10 **caractérisé en ce qu'**il comporte des moyens (CTC2d) pour retarder la mise en conduction dudit premier transistor MOSFET (20) tant que ledit deuxième transistor MOSFET (30) est commandé.

12. Convertisseur selon la revendication 11 **caractérisé en ce que** lesdits moyens (CTC2d) pour retarder la mise en conduction dudit premier transistor MOSFET (20) tant que ledit deuxième transistor MOSFET (30) est commandé comportent :
- un transistor MOSFET (50d), dit transistor de retardement,
- une résistance (53d) ayant une première borne reliée à la grille dudit transistor de roue libre (30) et une deuxième borne reliée à la grille dudit transistor de retardement (50d),
- une capacité (52d) ayant une première borne reliée à la grille dudit transistor de retardement (50d) et une deuxième borne reliée à la source dudit premier transistor MOSFET (20).

## Claims

1. A direct energy-transfer converter, comprising:
- a primary stage with at least one transformer primary winding and at least one controlled switch presenting conducting and nonconducting operating phases,
- a secondary stage including at least one secondary winding (10) of said transformer, and a synchronous rectifier (1), comprising:
• at least a first MOSFET transistor (20), known as the direct switch, when is self-regulated and conducting during the conducting phases of said controlled switch of the primary stage, known as the direct energy-transfer converter phases,
• at least a second MOSFET transistor (30), known as the freewheel switch, which is self-regulated and conducting during the nonconducting phases of said controlled switch of the primary stage, known as the freewheel phases,
- an output filter (5),
said converter comprises first self-regulated means which are triggered as a function of the voltage at the terminals of said at least one secondary winding (10), and applying to said second MOSFET transistor (30) a corresponding control voltage suitable to make the second MOSFET transistor (30) conduct, and
said first self-regulated means including a first charge-transfer circuit (CTC1) which is directly controlled by said voltage at the terminals of said secondary winding (10), to apply to said second MOSFET transistor (30) a controlled voltage which is more or less constant and supplied by an auxiliary voltage source (VAUX), **characterised in that** said at least one secondary winding is connected in parallel to the synchronous rectifier (1), and said charge-transfer circuit (CTC1) includes:
- a by-pass network which includes a capacitor (42) connected in series with a resistance (43), said resistance is connected to one end (14) of said at least one secondary winding (10), said circuit detecting the variation in the voltage induced by said at least one primary winding at the terminals of said at least one secondary winding (10).
- a bipolar transistor (40), known as the charge transistor, where the base of said bipolar transistor is controlled via said circuit, where the emitter of said bipolar transistor is connected to said auxiliary voltage source, and where the collector of said bipolar transistor is connected to the gate of the second MOSFET transistor (30).

2. A converter according to claim 1, **characterised in that** said auxiliary voltage source (VAUX) supplies a voltage (V60) which is more or less equal to the voltage of the at least one secondary winding (10) during the direct phase.

3. A converter according to claim 2, **characterised in that** said auxiliary voltage source (VAUX) comprises:
- a capacitance (60),
- a rectifier member (62) connected at one end in series with said capacitance (60) and at its other end to one end (15) of said at least one secondary winding (10), said rectifier element creating an auxiliary voltage which is more or less equal to the at least one secondary winding voltage (10) during the direct phase.

4. A converter according to claims 1 to 3, **characterised in that** the control voltage of said first MOSFET transistor (20) is obtained from the voltage at the terminals of said at least one secondary winding (10).

5. A converter according to any one of claims 1 to 3, **characterised in that** said converter, which includes second self-regulated means to apply a control voltage which will render conducting said first MOSFET transistor (20) in the direct phase, this control voltage is supplied by said auxiliary voltage source (VAUX) by means of a second charge-transfer circuit (CTC1d).

6. A converter according to claim 5, **characterised in that** a diode (41) is mounted in anti-parallel between the base and emitter of said bipolar charge transistor (40) .

7. A converter according to any of claims 1 to 6, **characterised in that** it includes means (CTC2) to anticipate the cut-off of said second MOSFET transistor (30) before switching into conduction of the first MOSFET transistor (20) in said direct phase.

8. A converter according to claim 7, **characterised in that** said means (CTC2) to anticipate the cut-off of said second MOSFET transistor (30) before the switching into conduction of said first MOSFET transistor (20) in said direct phase comprises:
- a by-pass network which includes a capacitor (52) connected in series with a resistance (53), where said resistance is connected to one end (14) of said at least one secondary winding (10), said circuit detecting the variation in the voltage induced by said primary winding at the terminals of said secondary winding (10).
- a bipolar transistor (50), known as the anticipation transistor, where the base of said bipolar transistor is controlled via said by-pass network, where the emitter of said bipolar transistor is connected to the drain of said first MOSFET transistor and the collector of the bipolar transistor is connected to the gate of said second MOSFET transistor.

9. A converter according to claim 8, **characterised in that** a diode (51) is connected in anti-parallel between the base and emitter of said anticipation bipolar transistor.

10. A converter according to claim 5, **characterised in that** said second charge-transfer circuit (CTC1d) comprises:
- a by-pass network which includes a capacitor (42d) connected in series with a resistance (43d), said resistance being connected to one end of said at least one secondary winding (10), said circuit detecting the variation in the voltage induced by said at least one primary winding at the terminals of said at least one secondary winding (10).
- a bipolar transistor (40d), known as the charge transistor, where the base of said bipolar transistor is controlled via said circuit, where the emitter of said bipolar transistor is connected to said auxiliary voltage source (VAUX) and where the collector of said bipolar transistor is connected to the gate of the first MOSFET transistor (20).

11. A converter according to any one of claims 1 to 10, **characterised in that** it includes the means (CTC2d) to delay the switching into conduction of said first MOSFET transistor (20) while said second MOSFET transistor (30) is controlled.

12. A converter according to claim 11, **characterised in that** said means (CTC2d) to delay the switching into conduction of the first MOSFET transistor (20) while the second MOSFET transistor (30) is controlled, comprises:
- a MOSFET transistor (50d), called delay transistor,
- a resistance (53d) with one end connected to the gate of said freewheel transistor (30) and its other end connected to the gate of said delay transistor (50d).
- a capacitance (52d) with one end connected to the gate of said delay transistor (50d) and a second end connected to the source of said first MOSFET transistor (20).

## Patentansprüche

1. Wandler mit direktem Energietransfer, umfassend:
- eine Primärstufe, die wenigstens eine Primärwicklung eines Transformators sowie wenigstens einen gesteuerten Schalter, der leitende und nichtleitende Betriebsphasen aufweist, umfaßt,
- eine Sekundärstufe mit wenigstens einer Sekundärwicklung (10) des Transformators sowie einem Synchrongleichrichter (1), umfassend:
- wenigstens einen ersten MOSFET-Transistor (20), sogenannten Direktschalter, der selbstgesteuert und während der leitenden Phasen des gesteuerten Schalters der Primärstufe, den sogenannten Phasen des direkten Energietransfers, leitend ist,
- wenigstens einen zweiten MOSFET-Transistor (30), sogenannten Freilaufschalter, der selbstgesteuert und während der nichtleitenden Phasen des gesteuerten Schalters der Primärstufe, den sogenannten Freilaufphasen, leitend ist,
- einen Ausgangsfilter (5),
wobei der Wandler erste selbstgesteuerte Mittel umfaßt, die in Abhängigkeit von der Spannung an den Anschlüssen der wenigstens einen Sekundärwicklung (10) ausgelöst werden und an den zweiten MOSFET-Transistor (30) eine entsprechende Steuerspannung anlegen, die geeignet ist, den zweiten Schalter (30) leitend zu machen,
die ersten selbstgesteuerten Mittel einen ersten Ladungstransferkreis (CTC1) umfassen, der direkt über die Spannung an den Anschlüssen der Sekundärwicklung (10) gesteuert wird, um an den zweiten Schalter (30) eine im wesentlichen konstante und durch eine Hilfsspannungsquelle (VAUX) gelieferte Steuerspannung anzulegen, **dadurch gekennzeichnet, daß** die wenigstens eine Sekundärwicklung mit dem Synchrongleichrichter (1) parallel geschaltet ist und der erste Ladungstransferkreis (CTC1) umfaßt:
- ein Abzweignetz, das einen Kondensator (42) umfaßt, der mit einem Widerstand (43) in Reihe geschaltet ist, wobei der Widerstand mit einem Ende (14) der wenigstens einen Sekundärwicklung (10) verbunden ist, wobei das Netz die Änderung der Spannung, die durch die wenigstens eine Primärwicklung an den Anschlüssen der wenigstens einen Sekundärwicklung (10) induziert wird, erfaßt,
- einen bipolaren Transistor (40), sogenannten Ladungstransistor, wobei die Basis des biopolaren Transistors über das Netz gesteuert wird, wobei der Emitter des bipolaren Transistors mit der Hilfsspannungsquelle verbunden ist und der Kollektor des biopolaren Transistors mit dem Gate des zweiten MOSFET-Transistors (30) verbunden ist.

2. Wandler nach Anspruch 1, **dadurch gekennzeichnet, daß** die Hilfsspannungsquelle (VAUX) eine Spannung (V60) liefert, die im wesentlichen gleich der Spannung der wenigstens einen Sekundärwicklung (10) während der direkten Phase ist.

3. Wandler nach Anspruch 2, **dadurch gekennzeichnet, daß** die Hilfsspannungsquelle (VAUX) umfaßt:
- ein C-Glied (60),
- ein Gleichrichterelement (62), das über sein erstes Ende mit dem C-Glied (60) und über sein zweites Ende mit einem Ende (15) der wenigstens einen Sekundärwicklung (10) in Reihe verbunden ist, wobei das Gleichrichterelement eine Hilfsspannung erzeugt, die im wesentlichen gleich der Spannung der wenigstens einen Sekundärwicklung (10) während der direkten Phase ist.

4. Wandler nach einem der Ansprüche 1 bis 3, **dadurch gekennzeichnet, daß** die Steuerspannung des ersten MOSFET-Transistors (20) mit Hilfe der Spannung an den Anschlüssen der wenigstens einen Sekundärwicklung (10) erhalten wird.

5. Wandler nach einem der Ansprüche 1 bis 3, **dadurch gekennzeichnet, daß** der Wandler zweite selbstgesteuerte Mittel umfaßt, um eine Steuerspannung anzulegen, die ermöglicht, den ersten MOSFET-Transistor (20) in direkter Phase leitend zu machen, diese Steuerspannung wird durch die Hilfsspannungsquelle (VAUX) mittels eines zweiten Ladungstransferkreises (CTC1d) geliefert.

6. Wandler nach Anspruch 5, **dadurch gekennzeichnet, daß** eine Diode (41) zwischen der Basis und dem Emitter des biopolaren Ladungstransistors (40) antiparallel geschaltet ist.

7. Wandler nach einem der Ansprüche 1 bis 6, **dadurch gekennzeichnet, daß** er Mittel (CTC2) umfaßt, um die Sperrung des zweiten MOSFET-Transistors (30) dem Einschalten des ersten MOSFET-Transistors (20) in den leitenden Zustand in der direkten Phase vorwegzunehmen.

8. Wandler nach Anspruch 7, **dadurch gekennzeichnet, daß** die Mittel (CTC2), um die Sperrung des zweiten MOSFET-Transistors (30) dem Einschalten des ersten MOSFET-Transistors (20) in den leitenden Zustand in der direkten Phase vorwegzunehmen, umfassen:
- ein Abzweignetz, das einen Kondensator (52) umfaßt, der mit einem Widerstand (53) in Reihe geschaltet ist, wobei der Widerstand mit einem Ende (14) der wenigstens einen Sekundärwicklung (10) verbunden ist, wobei das Netz die Änderung der Spannung, die durch die Primärwicklung an den Anschlüssen der Sekundärwicklung (10) induziert wird, erfaßt,
- einen bipolaren Transistor (50), sogenannten Vorwegnahme-Transistor, wobei die Basis des biopolaren Transistors über das Abzweignetz gesteuert wird, wobei der Emitter des bipolaren Transistors mit dem Drain des ersten Schalters verbunden ist und der Kollektor des biopolaren Transistors mit dem Gate des zweiten Schalters verbunden ist.

9. Wandler nach Anspruch 8, **dadurch gekennzeichnet, daß** eine Diode (51) zwischen der Basis und dem Emitter des bipolaren Vorwegnahme-Transistors antiparallel geschaltet ist.

10. Wandler nach Anspruch 4, **dadurch gekennzeichnet, daß** der zweite Ladungstransferkreis (CTC1d) umfaßt:
- ein Abzweignetz, das einen Kondensator (42d) umfaßt, der mit einem Widerstand (43d) in Reihe geschaltet ist, wobei der Widerstand mit einem Ende der wenigstens einen Sekundärwicklung (10) verbunden ist, wobei das Netz die Änderung der Spannung, die durch die wenigstens eine Primärwicklung an den Anschlüssen der wenigstens einen Sekundärwicklung (10) induziert wird, erfaßt,
- einen bipolaren Transistor (40d), sogenannten Ladungstransistor, wobei die Basis des biopolaren Transistors über das Netz gesteuert wird, wobei der Emitter des bipolaren Transistors mit der Hilfsspannungsquelle (VAUX) verbunden ist und der Kollektor des biopolaren Transistors mit dem Gate des ersten MOSFET-Transistors (20) verbunden ist.

11. Wandler nach einem der Ansprüche 1 bis 10, **dadurch gekennzeichnet, daß** er Mittel (CTC2d) umfaßt, um das Einschalten des ersten MOSFET-Transistors (20) in den leitenden Zustand solange zu verzögern wie der zweite MOSFET-Transistor (30) gesteuert wird.

12. Wandler nach Anspruch 11, **dadurch gekennzeichnet, daß** die Mittel (CTC2d), um das Einschalten des ersten MOSFET-Transistors (20) in den leitenden Zustand solange zu verzögern wie der zweite MOSFET-Transistor (30) gesteuert wird, umfassen:
- einen MOSFET-Transistor (50d), sogenannten Verzögerungstransistor,
- einen Widerstand (53d), der einen ersten Anschluß, welcher mit dem Gate des Freilauftransistors (30) verbunden ist, und einen zweiten Anschluß, der mit dem Gate des Verzögerungstransistors (50d) verbunden ist, aufweist,
- ein C-Glied (52d), das einen ersten Anschluß, der mit dem Gate des Verzögerungstransistors (50d) verbunden ist, und einen zweiten Anschluß, der mit der Source des ersten MOSFET-Transistors (20) verbunden ist, aufweist.
